(19) Europäisches Patentamt
European Patent Office
Office européen des brevets

(11) **EP 3 162 375 B1**

(12) **EUROPEAN PATENT SPECIFICATION**

(45) Date of publication and mention
of the grant of the patent:
**06.11.2019 Bulletin 2019/45**

(51) Int Cl.:
*A61K 36/539* (2006.01)    *A61K 36/536* (2006.01)
*A61K 8/97* (2017.01)    *A61P 17/00* (2006.01)
*A61Q 19/02* (2006.01)    *A61K 8/11* (2006.01)

(21) Application number: **15814888.2**

(22) Date of filing: **26.06.2015**

(86) International application number:
**PCT/KR2015/006563**

(87) International publication number:
**WO 2016/003120 (07.01.2016 Gazette 2016/01)**

(54) **WHITENING COMPOSITION COMPRISING SCUTELLARIA ALPINA EXTRACT**

BLEICHUNGSZUSAMMENSETZUNG MIT SCUTELLARIA-ALPINA-EXTRAKT

COMPOSITION DE BLANCHIMENT COMPRENANT UN EXTRAIT DE SCUTELLARIA ALPINA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2014 KR 20140080478**

(43) Date of publication of application:
**03.05.2017 Bulletin 2017/18**

(73) Proprietor: **Amorepacific Corporation**
**Seoul 140-777 (KR)**

(72) Inventors:
• **PARK, Pil Joon**
**Yongin-si**
**Gyeonggi-do 446-729 (KR)**
• **LEE, So Hee**
**Yongin-si**
**Gyeonggi-do 446-729 (KR)**
• **CHO, Eun Gyung**
**Yongin-si**
**Gyeonggi-do 446-729 (KR)**
• **LEE, Tae Ryong**
**Yongin-si**
**Gyeonggi-do 446-729 (KR)**

(74) Representative: **Agasse, Stéphane et al**
**Cabinet GERMAIN & MAUREAU**
**B.P. 6153**
**69466 Lyon Cedex 06 (FR)**

(56) References cited:
**WO-A2-2008/121315    CN-A- 103 301 377**

• **LI X: "Baicalein inhibits melanogenesis through activation of the ERK signaling pathway", INTERNATIONAL JOURNAL OF MOLECULAR MEDICINE, vol. 25, no. 6, 21 April 2010 (2010-04-21) , XP055441367, GR ISSN: 1107-3756, DOI: 10.3892/ijmm_00000423**
• **KIM NA-YOUNG: "Effect of antioxdation and inhibition of melanogenesis from Scutellaria baicalensis Extract", AESTHET KOR J, COSMETOL, vol. 12, no. 1, 15 February 2014 (2014-02-15), pages 41-47, XP055441634,**
• **Brajesh N Vaidya ET AL: "Antioxidant Capacity of Fresh and Dry Leaf Extracts of Sixteen Scutellaria Species", Journal of Medicinally Active Plants, vol. 2, no. 3-4 1 January 2014 (2014-01-01), pages 42-49, XP055421557, DOI: 10.7275/R5J9649K Retrieved from the Internet: URL:http://scholarworks.umass.edu/cgi/view content.cgi?article=1063&context=jmap**
• **'Alpaflor Scutellaria ao. Datasheet', [Online] 2010, pages 1 - 7, XP055250923 Retrieved from the Internet: <URL:http://www.centerchem.com/Products/alp aflor-scuttelaria-ao > </Product>>**
• **SCHAEFER, K.: 'Men's skin care formulating concepts' 04 May 2010, pages 1 - 1, XP055233528 Retrieved from the Internet: <URL:http://www.cosmeticsandtoiletries.com/ formulating/category/menscare/92809404 .html >**

- KIKUCHI, Y. ET AL.: 'Studies on the constituents of Scutellaria species. XIV. On the constituents of the roots and the leaves of Scutellaria alpina L.' CHEMICAL AND PHARMACEUTICAL BULLETIN vol. 39, no. 1, 1991, pages 199 - 201, XP055250922

- BOZOV, P. I. ET AL.: 'Scutalpin A, a neo-clerodane diterpene from Scutellaria alpina' PHYTOCHEMISTRY vol. 34, no. 2, 1993, ISSN 0031-9422 pages 453 - 456, XP026632059

**Description**

[Background Art]

The present disclosure relates to a composition containing a *Scutellaria alpina* extract.

[Background Art]

Melanin is a pigment that determines skin color in humans. The skin color is determined by the amount and distribution of melanin. The cells producing melanin are derived from melanocytes located in the bottom layer of the epidermis and are lost after moving toward the keratinous layer as a result of skin metabolism. The number of melanocytes is almost constant regardless of skin color but the difference in the amount, type and distribution of the produced melanin results in different skin colors.

In the skin, tyrosine is converted to DOPA by the enzyme called tyrosinase, which is finally turned into the blackish brown polymer melanin through a series of oxidation processes.

Melanin is produced by melanocytes located in the basal layer of the skin. It is known that the melanin production is promoted by stimuli such as UV, inflammation, etc. Therefore, by reducing external stimulation, interrupting signal transduction, suppressing the synthesis of the melanin-producing enzyme tyrosinase or inhibiting its activity, the production of melanin can be decreased.

At present, it is known that kojic acid, hydroquinone, arbutin, azelaic acid, aloesin, 4-butylresorcinol, resveratrol, ceramide, sphingosine-1-phosphate, sphingosylphosphorylcholine, etc. can control the melanin production by promoting the degradation of tyrosinase or by regulating its glycosylation. However, their utility is not so high because of unsatisfactory skin whitening effect, low stability and skin irritation. Accordingly, there is a need of a substance which exhibits superior skin whitening effect with few side effects.

[References of Related Art]

[Patent Document]

KR10-1111533 B1.

**[Disclosure]**

[Technical Problem]

The present disclosure is directed to providing a composition having a superior skin whitening effect.

[Technical Solution]

In an aspect, the present disclosure provides a composition for skin whitening, which contains an extract of *Scutellaria alpina* belonging to the genus *Scutellaria* as an active ingredient.

[Advantageous Effects]

The composition for skin whitening of the present disclosure, which contains a *Scutellaria alpina* extract as an active ingredient, exhibits skin whitening effect by reducing melanin production and inhibiting tyrosinase activity while having stability without negatively affecting cells. Accordingly, it can be widely used for a composition for extremal application to skin, a cosmetic composition and a pharmaceutical composition.

[Brief Description of Drawings]

Fig. 1 shows a graph showing the result of testing the decrease in melanin content by a *Scutellaria alpina* extract according to the present disclosure as well as a photograph taken before the measurement.
Fig. 2 shows a result of testing the decrease in tyrosinase activity by a *Scutellaria alpina* extract according to the present disclosure.

[Best Mode for Carrying Out Invention]

Hereinafter, specific exemplary embodiments of the present disclosure will be described in detail, so that those of ordinary skill in the art to which the present disclosure belongs can easily carry out the present disclosure.

In an aspect, the present disclosure may relate to a composition containing a *Scutellaria alpina* extract as an active ingredient.

In an aspect of the present disclosure, the composition may be a composition for skin whitening.

In an aspect of the present disclosure, the composition according to the present disclosure may be a pharmaceutical, food or cosmetic composition. And specifically, in an aspect of the present disclosure, the composition according to the present disclosure may be a composition for extremal application to skin.

In an aspect, the present disclosure may relate to a method for skin whitening, which includes administering a *Scutellaria alpina* extract to a subject in need of skin whitening. Specifically, the administration may be achieved by an administration method described in the present disclosure.

In an aspect, the present disclosure may relate to a use of a *Scutellaria alpina* extract for skin whitening.

In an aspect, the present disclosure may relate to a *Scutellaria alpina* extract for use in skin whitening.

In an aspect, the present disclosure may relate to a composition for reducing melanin, which contains a *Scutellaria alpina* extract as an active ingredient.

In an aspect, the present disclosure may relate to a method for reducing melanin, which includes administering a *Scutellaria alpina* extract to a subject in need of melanin reduction. Specifically, the administration may be achieved by an administration method described in the present disclosure.

In an aspect, the present disclosure may relate to a use of a *Scutellaria alpina* extract for melanin reduction.

In an aspect, the present disclosure may relate to a *Scutellaria alpina* extract for use in melanin reduction.

In an aspect, the present disclosure may relate to a composition for suppressing melanin production, which contains a *Scutellaria alpina* extract as an active ingredient.

In an aspect, the present disclosure may relate to a method for suppressing melanin production, which includes administering a *Scutellaria alpina* extract to a subject in need of suppression of melanin production. Specifically, the administration may be achieved by an administration method described in the present disclosure.

In an aspect, the present disclosure may relate to a use of a *Scutellaria alpina* extract for suppressing melanin production.

In an aspect, the present disclosure may relate to a *Scutellaria alpina* extract for use in suppression of melanin production.

In the present disclosure, "melanin reduction" may mean reducing the content of melanin already existing in cells, etc., and "suppression of melanin production" may mean suppressing the new production of melanin in cells, etc.

In an aspect, the present disclosure may relate to a composition for inhibiting tyrosinase activity, which contains a *Scutellaria alpina* extract as an active ingredient.

In an aspect, the present disclosure may relate to a method for inhibiting tyrosinase activity, which includes administering a *Scutellaria alpina* extract to a subject in need of inhibition of tyrosinase activity.

In an aspect, the present disclosure may relate to a use of a *Scutellaria alpina* extract for inhibiting tyrosinase activity.

In an aspect, the present disclosure may relate to a *Scutellaria alpina* extract for use in inhibition of tyrosinase activity.

In an aspect, the present disclosure may relate to a composition for suppressing pigmentation, which contains a *Scutellaria alpina* extract as an active ingredient.

In an aspect, the present disclosure may relate to a composition for treating, improving or preventing pigmentation disorder, which contains a *Scutellaria alpina* extract as an active ingredient. Specifically, in an aspect, the present disclosure may relate to a pharmaceutical composition for treating or preventing pigmentation disorder or a food composition for improving or preventing pigmentation disorder, which contains a *Scutellaria alpina* extract as an active ingredient.

In an aspect, the present disclosure may relate to a method for treating, improving or preventing pigmentation disorder, which includes administering a *Scutellaria alpina* extract to a subject in need of treatment, improvement or prevention of pigmentation disorder. Specifically, the administration may be achieved by an administration method described in the present disclosure.

In an aspect, the present disclosure may relate to a use of a *Scutellaria alpina* extract for treating, improving or preventing pigmentation disorder.

In an aspect, the present disclosure may relate to a *Scutellaria alpina* extract for use in treatment, improvement or prevention of pigmentation disorder.

In an aspect of the present disclosure, the term pigmentation disorder is used in a broad concept, including all the pigmentation disorders caused by various causes. Specifically, it may refer to pigmentation disorder occurring in skin, but is not limited thereto. Specifically, in an aspect of the present disclosure, the pigmentation disorder may include senile spots, inflammatory pigmentation disorder, freckles, acquired dermal melanosis, hyperpigmentation, etc., but is not limited thereto.

In the present disclosure, the term "pigmentation" or "hyperpigmentation" refers to a skin damage characterized by actual or perceived, excessively dark skin color. The skin damage may be actual and dark skin areas may appear due to such conditions as aging, excessive exposure to sunlight or diseases. The dark skin area may be in the form of spots, blotches or relatively large dark areas. The skin damage may also be one perceived by individuals who want to have light skin.

Accordingly, the composition of the present disclosure is useful for various types of skin pigmentation such as melanoma, for example, in removing pigmented dark patches on the face or other parts of the body or inducing spontaneous whitening of pigmented skin. Typically, the dark skin damage is caused by increased melanin level. The composition according to the present disclosure may be used to treat hyperpigmentation, i.e., to lighten the dark skin or prevent hyperpigmentation, or to prevent skin darkening by reducing or preventing excessive melanin production.

In an aspect of the present disclosure, the concentration of the *Scutellaria alpina* extract in the composition containing the same may be 0.1-10 ppm (w/w) based on the total weight of the composition, although not being limited thereto. Specifically, in an aspect of the present disclosure, the concentration of the *Scutellaria alpina* extract in the composition containing the same may be 0.01 ppm or higher, 0.1 ppm or higher, 0.5 ppm or higher, 0.6 ppm or higher, 0.7 ppm or higher, 0.8 ppm or higher, 0.9 ppm or higher, 1.0 ppm or higher, 1.1 ppm or higher, 1.2 ppm or higher, 1.3 ppm or higher, 1.4 ppm or higher, 1.5 ppm or higher, 1.6 ppm or higher, 1.7 ppm or higher, 1.8 ppm or higher, 1.9 ppm or higher, 2.0 ppm or higher, 2.1 ppm or higher, 2.2 ppm or higher, 2.3 ppm or higher, 2.4 ppm or higher, 2.5 ppm or higher, 2.6 ppm or higher, 2.7 ppm or higher, 2.8 ppm or higher, 2.9 ppm or higher, 3 ppm or higher, 3.2 ppm or higher, 3.4 ppm or higher, 3.6 ppm or higher, 3.8 ppm or higher, 4.0 ppm or higher, 4.5 ppm or higher, 5.0 ppm or higher, 5.5 ppm or higher, 6.0 ppm or higher, 7.0 ppm or higher, 8.0 ppm or higher, 9.0 ppm or higher, 10.0 ppm or higher, 50 ppm or higher, 100 ppm or higher, 500 ppm or higher or 1000 ppm or higher, although not being limited thereto, and may be 2000 ppm or lower, 1000 ppm or lower, 500 ppm or lower, 100 ppm or lower, 50 ppm or lower, 15.0 ppm or lower, 10.0 ppm or lower, 8.0 ppm or lower, 6.0 ppm or lower, 5.5 ppm or lower, 5.0 ppm or lower, 4.5 ppm or lower, 4.0 ppm or lower, 3.8 ppm or lower, 3.6 ppm or lower, 3.4 ppm or lower, 3.2 ppm or lower, 3.0 ppm or lower, 2.8 ppm or lower, 2.6 ppm or lower, 2.4 ppm or lower, 2.2 ppm or lower, 2.0 ppm or lower, 1.8 ppm or lower, 1.6 ppm or lower, 1.4 ppm or lower, 1.2 ppm or lower, 1.0 ppm or lower, 0.8pm or lower, 0.6 ppm or lower, 0.4 ppm or lower or 0.2 ppm or lower, based on the total weight of the composition. The concentration of the extract is in ppm (w/w) unit.

In an aspect of the present disclosure, the *Scutellaria alpina* may be one or more selected from a group consisting of the leaf, flower, stem and root of the plant *Scutellaria alpina.* Specifically, it may be a mixture of the flower, leaf and stem of the plant *Scutellaria alpina.*

In an aspect of the present disclosure, the *Scutellaria alpina* extract may be prepared by a method including (1) a step of extracting *Scutellaria alpina* with water, an organic solvent or a combination thereof.

In an aspect of the present disclosure, the method may further include, prior to the step (1), a step of processing *Scutellaria alpina.* Specifically, the processing may be drying and pulverizing *Scutellaria alpina.* However, without being limited thereto, any processing for facilitating the extraction is included. Specifically, the drying may be sunlight drying, hot air drying, evaporation drying, spray drying or freeze drying, more specifically hot air drying. On the other hand, in an aspect of the present disclosure, live *Scutellaria alpina* may be extracted as it is without any processing.

In an aspect of the present disclosure, the method may further include a step of removing the solvent by distillation following the extraction. Specifically, the distillation may be vacuum distillation.

In an aspect of the present disclosure, the method may further include a step of adding one or more of glycerin and a preservative to the resulting concentrate following the distillation.

In an aspect of the present disclosure, the method may further include a step of filtering following the solvent removal or following the addition of one or more of glycerin and a preservative.

In an aspect of the present disclosure, the *Scutellaria alpina* extract may be an extract of one or more selected from a group consisting of water, an organic solvent and a mixture thereof. Specifically, in an aspect of the present disclosure, the organic solvent may be one or more selected from a group consisting of a $C_1$-$C_6$ lower alcohol, butylene glycol and propylene glycol. More specifically, the lower alcohol may be ethanol.

Melanocytes used in the present disclosure may be the melanocytes disclosed in PCT/KR2012/007815 or Korean Patent Application No. 10-2011-0099728 (keratinocyte-adapted melanocytes, KaMC). The melanocytes used in the present disclosure may be obtained according to the method and procedure disclosed in the references and may also be cultured according to the method disclosed in the references. The melanocytes were deposited in the Korean Collection for Type Culture (KCTC) of Korea Research Institute of Bioscience and Biotechnology on Sep. 14, 2011 under the Budapest Treaty and were given the accession number KCTC 12015BP.

In the present disclosure, "skin" refers to the organ covering the surface of an organism. It is composed of the epidermis, the dermis and the subcutaneous fat layer and is used in the broadest concept, including not only the tissues covering the face or the entire outer part of the body but also the scalp and hair.

In the present disclosure, "*Scutellaria alpina*" refers to a plant in the genus *Scutellaria* of the family *Lamiaceae*. It is about 10-30 cm tall. It has short hairs on the whole plant and the stems are square and prostrate-ascending. The flowers are blue-violet or purple, 2.4-3 cm long. *Scutellaria alpine* prefer rocky areas in high calcareous mountains at elevation of 1400-2500 m above sea level.

In the present disclosure, "extract" refers to a substance extracted from a natural product, regardless of the extraction method, extraction solvent, extracted components or the type of the extract. It is used in a broad concept, including any substance that can be obtained by processing or treatment of a substance extracted from a natural product. Specifically, the processing or treatment may be fermenting or enzymatically treating the extract. Accordingly, in the present disclosure, the extract is used in a broad concept, including a fermentation product, a concentrate and a dried product. Specifically, in the present disclosure, the extract may be a fermentation product.

In the present disclosure, "*Scutellaria alpina* extract" includes any substance obtained by extracting *Scutellaria alpina,* regardless of the extraction method, extraction solvent, extracted components or the type of the extract. It includes a substance obtained by an extraction method including a process treating with heat, an acid, a base, an enzyme, etc. and is used in a broad concept, including any substance that can be obtained by processing or treatment of a substance extracted from *Scutellaria alpina.* Specifically, the processing or treatment may be fermenting or enzymatically treating the *Scutellaria alpina* extract. Accordingly, the *Scutellaria alpina* extract of the present disclosure may be a fermentation product.

In an aspect of the present disclosure, "*Scutellaria alpina*" may be an extract, live *Scutellaria alpina,* a pulverization product of live *Scutellaria alpina,* a dried product of live *Scutellaria alpina,* a dried pulverization product of live *Scutellaria alpina* or a fermentation product *Scutellaria alpina,* although not being limited thereto. And, the *Scutellaria alpina* used in the present disclosure is not limited in the way how it is obtained. It may be either cultivated or purchased commercially. Also, all or part of its areal part or root part may be used. More specifically, one or more selected from a group consisting of the leaf, stem, root and flower of the plant *Scutellaria alpina* may be used. The *Scutellaria alpina* of the present disclosure is not necessarily dried and may be in any form as long as the active ingredients of *Scutellaria alpina* can be adequately extracted.

In an aspect of the present disclosure, the water includes distilled water or purified water, and the organic solvent includes one or more selected from a group consisting of an alcohol such as a $C_1$-$C_5$ lower alcohol, acetone, ether, ethyl acetate, diethyl ether, methyl ethyl ketone and chloroform, although not being limited thereto.

In an aspect of the present disclosure, the *Scutellaria alpina* extract may include a $C_1$-$C_6$ alcohol extract of *Scutellaria alpina.* Specifically, the alcohol may be methanol or ethanol.

In an aspect of the present disclosure, the *Scutellaria alpina* extract may be obtained by a method including a step of extracting *Scutellaria alpina* with water, an organic solvent or a mixture thereof.

In an aspect of the present disclosure, the *Scutellaria alpina* extract may be a crude extract of a solvent selected from a group consisting of water, an organic solvent and a combination thereof. The organic solvent may be a $C_1$-$C_6$ alcohol. Specifically, the $C_1$-$C_6$ alcohol may be methanol or ethanol. In an aspect of the present disclosure, when the *Scutellaria alpina* is extracted with a solvent, the solvent may be added in an amount of about 5-15 times that of the *Scutellaria alpina.* Specifically, solvent may be added in an amount of about 10 times that of the *Scutellaria alpina,* although not being limited thereto.

In an aspect of the present disclosure, the extraction may be performed by hot water extraction, ethanol extraction, heating extraction, cold extraction, reflux extraction, reflux condensation extraction, ultrasonic extraction, etc. Any extraction method known to those skilled in the art may be used without limitation. Specifically, the extraction may be performed by hot water extraction or ethanol extraction.

In an aspect of the present disclosure, the extraction may be performed at room temperature. However, for more effective extraction, it may be performed at elevated temperatures. The extraction may be performed specifically at about 40-100 °C, more specifically at about 80 °C, although not being limited thereto. The extraction may be performed for about 2-14 hours, specifically for about 8-14 hours, more specifically for about 11-13 hours, most specifically for 12 hours. However, the extraction time is not limited thereto and may vary depending on such conditions as extraction solvent, extraction temperature, etc. The extraction may be performed more than once in order to obtain a larger amount of the active ingredients. The extraction may be performed continuously specifically 1-5 times, more specifically 3 times, and the resulting extracts may be combined for further use.

In an aspect of the present disclosure, the *Scutellaria alpina* extract may include the crude extract of *Scutellaria alpina* as described above and may also include a soluble fraction of an organic solvent with low polarity, which is obtained by further extracting the crude extract with the organic solvent. In an aspect of the present disclosure, the organic solvent may be hexane, methylene chloride, ethyl acetate, n-butanol, etc., although not being limited thereto. The extract or the soluble fraction of the extract may be used as it is, after being filtered and then concentrated, or after being concentrated and dried.

In an aspect of the present disclosure, the drying may be evaporation drying, spray drying or freeze drying.

Specifically, the freeze drying may be performed at - 50 to -70 °C for 3-4 days.

Formulations of the cosmetic composition according to an aspect the present disclosure are not particularly limited but may be selected properly depending on purposes. For example, the composition may be prepared into one or more formulation selected from a group consisting of skin lotion, skin softener, skin toner, astringent lotion, milk lotion, moisturizing lotion, nourishing lotion, massage cream, nourishing cream, moisturizing cream, hand cream, foundation, essence, nourishing essence, pack, soap, cleansing foam, cleansing lotion, cleansing cream, body lotion and body cleanser, although not being limited thereto.

When the cosmetic composition according to the present disclosure is formulated as paste, cream or gel, animal fiber, plant fiber, wax, paraffin, starch, tragacanth, cellulose derivatives, polyethylene glycol, silicone, bentonite, silica, talc, zinc oxide, etc. may be used as a carrier component.

When the cosmetic composition according to the present disclosure is formulated as powder or spray, lactose, talc, silica, aluminum hydroxide, calcium silicate or polyamide powder may be used as a carrier component. In particular, when it is formulated as spray, it may further contain a propellant such as chlorofluorohydrocarbon, propane/butane or dimethyl ether.

When the cosmetic composition according to the present disclosure is formulated as solution or emulsion, a solvent, a solubilizer or an emulsifier may be used as a carrier component. For example, water, ethanol, isoproanol, ethyl carbonate, ethyl acetate, benzyl alcohol, benzyl benzoate, propylene glycol, 1,3-butylglycol oil, glycerol fatty ester, or fatty acid ester of polyethylene glycol or sorbitan may be used.

When the cosmetic composition according to the present disclosure is formulated as suspension, a diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier component.

When the cosmetic composition according to the present disclosure is formulated as surfactant-containing cleanser, fatty alcohol sulfate, fatty alcohol ether sulfate, sulfosuccinic acid monoester, isethionate, imidazolinium derivatives, methyl taurate, sarcosinate, fatty acid amide ether sulfate, alkyl amidobetaine, fatty alcohol, fatty acid glyceride, fatty acid diethanolamide, vegetable oil, lanolin derivatives, ethoxylated glycerol fatty acid ester, etc. may be used as a carrier component.

The cosmetic composition according to the present disclosure may further contain a functional additive and ingredients commonly used in a cosmetic composition, in addition to the *Scutellaria alpina* extract. The functional additive may include those selected from a group consisting of water-soluble vitamins, oil-soluble vitamins, polypeptides, polysaccharides, sphingolipids and seaweed extract.

If necessary, the cosmetic composition of the present disclosure may further contain, in addition to the functional additive, the ingredients commonly used in a cosmetic composition. Examples of the further contained ingredients may include oils, fats, humectants, emollients, surfactants, organic and inorganic pigments, organic powders, UV absorbers, preservatives, sterilizers, antioxidants, plant extracts, pH control agents, alcohols, colorants, flavors, blood circulation promoters, coolants, deodorants antiperspirants, purified water, etc.

The present disclosure also relates to a composition for external application to skin, which contains a *Scutellaria alpina* extract as an active ingredient. The composition for external application to skin refers to any type of composition that can be applied from outside the skin and various types of cosmetics may be included therein.

The pharmaceutical composition according to the present disclosure may be in the form of various formulations, including oral or parenteral. When the composition is formulated, a commonly used diluent or excipient such as a filler, an extender, a binder, a wetting agent, a disintegrant, a surfactant, etc. is used. Solid formulations for oral administration include a tablet, a pill, a powder, a granule, a soft or hard capsule, etc., and these solid formulations may be prepared by mixing with at least one excipient such as starch, calcium carbonate, sucrose, lactose, gelatin, etc. In addition to the simple excipient, a lubricant such as magnesium stearate, talc, etc. may also be used. Examples of liquid formulations for oral administration include a suspension, a liquid medicine for internal use, an emulsion, a syrup, etc. In addition to a commonly used simple diluent such as water and liquid paraffin, various excipients such as a humectant, a sweetener, an aromatic, a preservative, etc. may also be contained. Formulations for parenteral administration include a sterilized aqueous solution, a non-aqueous solution, a suspension, an emulsion, a lyophilized product and a suppository. The non-aqueous solution or suspension may contain propylene glycol, polyethylene glycol, vegetable oil such as olive oil, injectable ester such as ethyl oleate, etc. As a base of the suppository, witepsol, macrogol, tween 61, cocoa butter, laurin butter, glycerogelatin, etc. may be used.

The composition of the present disclosure may contain a pharmaceutically acceptable salt of the active ingredient, and may be used alone or in combination with another pharmaceutically active compound(s). The salt is not particularly limited as long as it is pharmaceutically acceptable. For example, a salt of hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, naphthalenesulfonic acid, etc. may be used.

The composition of the present disclosure may be administered parenterally or orally depending on the desired purposes. A daily dose of 0.1-500 mg, specifically 1-100 mg, per kg body weight may be administered once or several times a day. The administration dose for a particular patient may be changed depending on the body weight, age, sex and health conditions of the patient, diet, administration time, administration method, rate of excretion, severity of disease, and so forth.

The pharmaceutical composition according to the present disclosure may be formulated into any pharmaceutically appropriate form including oral formulations such as powder, granule, tablet, soft or hard capsule, suspension, emulsion, syrup, aerosol, etc., agents for external application to skin such as ointment, cream, etc., suppository, injection, sterile solution for injection, etc. according to commonly employed methods. Specifically, it may be formulated into an injection or an agent for external application to skin.

The composition according to the present disclosure may be administered to mammals including rat, mouse, cattle, human, etc. via various routes including parenteral and oral routes. All types of application may be expected. For example, it may be administered orally, transdermally, rectally, intravenously, intramuscularly, subcutaneously, intrauterinally, or intracerebroventricularly.

The composition according to the present disclosure may be administered via various routes that can be easily selected by those skilled in the art. In particular, the pharmaceutical composition according to the present disclosure may be applied on skin as an agent for external application to skin.

In an aspect of the present disclosure, the food composition may be a health functional food composition.

Formulations of the food composition according to the present disclosure are not particularly limited. For example, it may be formulated into a tablet, a granule, a powder, a liquid such as a drink, a caramel, a gel, a bar, etc. The food composition of each formulation may contain, in addition to the active ingredient, ingredients commonly used in the art that can be selected by those skilled in the art without difficulty. In this case, a synergic effect may be achieved.

In the food composition according to the present disclosure, determination of the administration dose of the active ingredient is within the level of those skilled in the art. For example, a daily dosage may be 0.1-5000 mg/kg/day, more specifically, 50-500 mg/kg/day. However, without being limited thereto, the administration dose may vary depending on various factors such as the age and health conditions of the subject, presence of complication(s), etc.

For example, the food composition according to the present disclosure may be various foods such as chewing gum, caramel, candy, ices, confectionery, etc., drinks such as soft drink, mineral water, alcohol beverage, etc. or health functional foods including vitamin, mineral, etc.

In addition, the food composition according to the present disclosure may contain various nutrients, vitamins, minerals (electrolytes), flavors including synthetic and natural flavors, colorants, extenders (cheese, chocolate, etc.), pectic acid and salts thereof, alginic acid and salts thereof, organic acids, protective colloidal thickeners, pH control agents, stabilizers, preservatives, glycerin, alcohols, carbonating agents used in carbonated drinks, etc. And, the functional food composition of the present disclosure may contain pulps used to prepare natural fruit juice, fruit juice drinks and vegetable drinks. These ingredients may be used independently or in combination. The content of these additives is of no great importance. Usually, they are contained within a range of about 0-20 parts by weight based on 100 parts by weight of the composition of the present disclosure.

Hereinafter, the present disclosure will be described in detail through examples and test examples. However, the following examples and test examples are for illustrative purposes only and it will be apparent to those of ordinary skill in the art that the scope of the present disclosure is not limited by the examples and test examples.

[Example 1] Preparation of *Scutellaria alpina* extract

The flower, leaf and stem of *Scutellaria alpina* were harvested, hot air dried together and pulverized into powder. The resulting powder was extracted with an ethanol/water solution and then the ethanol was removed through vacuum distillation. After adding glycerin and a preservative to the resulting concentrate, a *Scutellaria alpina* extract was obtained by filtration. A *Scutellaria alpina* extract (Alpaflor® Scutellaria AO) prepared according to the above-described method was purchased from DSM Nutritional Products Ltd. (4002 Basel, Switzerland) for use in the following test examples.

[Test Example 1] Measurement of melanin

(1) Cell culturing

Melanocytes (KaMC) (KCTC12015BP) deposited in the Korean Collection for Type Culture (KCTC) were cultured in M-254 medium (Gibco BRL, NY, USA) supplemented with Human Melanocyte Growth Supplement (HMGS; Gibco BRL, NY, USA) on a 6-well plate, while changing the medium every other day, in a 5% $CO_2$ incubator at 37 °C until the cells occupied 95% of the plate area (95% confluence).

(2) Treatment with extract

When the density of the melanocytes on the 6-well plate reached about 80% (~80% confluence), they were incubated for 5 days with 10 nM of a pigmentation-inducing substance (human endothelin 1, EDN1; ProSpec Bio Co., USA) and the *Scutellaria alpina* extract of Example 1 of different concentrations (0, 0.1, 1, 2 and 5 ppm (w/w)). The cells not treated with the pigmentation-inducing substance and the *Scutellaria alpina* extract of Example 1 were used as a control group. The pigmentation-inducing substance (EDN1) is a protein encoded by the human EDN1 gene.

(3) Recovery of cells and quantification of proteins

The melanocytes (KaMC) cultured on the 6-well plate were added to 400 $\mu$L of an Accutase solution (Millipore, CA, USA). After agitation for 1 minute, a total of 1 mL of a cell-containing medium was recovered by adding 600 $\mu$L of M-254 medium. Then, after centrifugation at 12,000 rpm for 15 minutes, the supernatant was discarded and 20 $\mu$L of MCL lysis buffer (Sigma-Aldrich, St. Louis, USA) was added to the remaining pellets. After mixing well and centrifuging again at 12,000 rpm for 15 minutes, the supernatant and the cell debris were separated.

After adding 1 $\mu$L of the separated supernatant to a 96-well plate, distilled water was added to make the final volume 25 $\mu$L. Then, the quantity of proteins was determined at 562 nm using the BCA protein assay kit (Thermo Scientific, IL, USA). The result is shown in Table 1.

[Table 1]

| Sample | | Mean ($\mu$g/$\mu$L) | Standard deviation |
|---|---|---|---|
| Pigmentation-inducing substance (endothelin 1) (nM) | *Scutellaria alpina* extract (ppm) | | |
| 0 | 0 | 4.2949878 | 0.1810235 |
| 10 | 0 | 3.9254528 | 0.1584871 |
| | 0.1 | 4.012156 | 0.1932569 |
| | 1 | 4.114588 | 0.150455 |
| | 2 | 3.856595 | 0.19502161 |
| | 5 | 4.15516 | 0.1329656 |

(4) Melanin assay

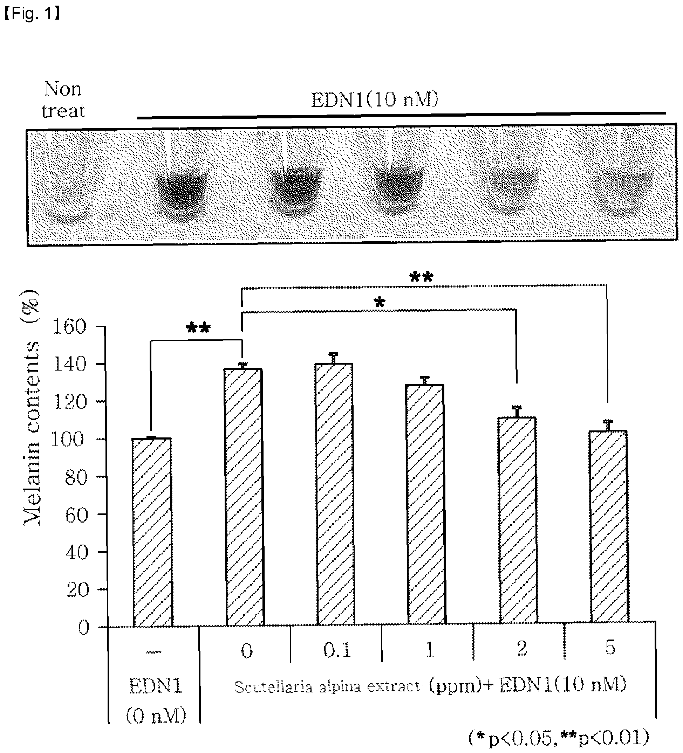

The cell debris separated in (3) of Test Example 1 were lysed at 55 °C for 15 minutes after adding 70 $\mu$L of 1 N NaOH (Sigma-Aldrich, St. Louis, USA) and absorbance was measured at 475 nm after transferring to two 96-well plates, 30 $\mu$L each. Fig. 1 shows a photograph taken before the measurement and a result of calculating the melanin content from the quantitated protein amount according to Equation 1.

[Equation 1]

$$\text{Melanin content (\%)} = (\text{Absorbance} / \text{Total protein}) \times 100$$

As seen from Fig. 1, when the cells were treated with the *Scutellaria alpina* extract of the present disclosure, the melanin content which had been increased by the pigmentation-inducing substance (EDN1) was decreased. In particular, when 5 ppm of the *Scutellaria alpina* extract was treated, the melanin content decreased to about 102%, comparable to that of the control group. This result could also be perceived visually before the absorbance was measured. That is, the color of the test tube was brighter as the concentration of the *Scutellaria alpina* extract increased. When the concentration was 5 ppm, the test tube was as clear as the control group. Accordingly, it was confirmed that the *Scutellaria alpina* extract of the present disclosure exhibits an effect of reducing the melanin content in cells and thus it can exhibit the effect of whitening skin or treating pigmentation.

[Test Example 2] Inhibition of tyrosinase activity

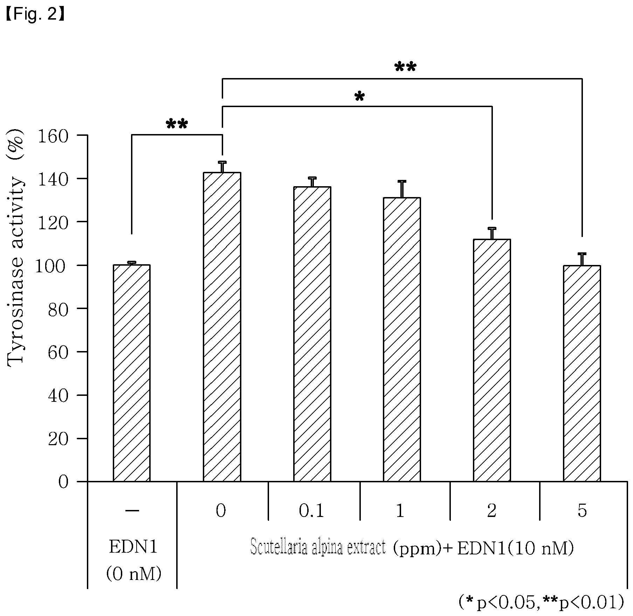

Based on the result of protein quantification in (3) of Test Example 1, proteins of the amount contained in the supernatant, i.e. 20 μg, was added to a 96-well plate and PBS (phosphate-buffered saline, without CaCl & MgCl, Welgene, Korea) was added to make the final volume 100 μL. Then, 100 μL of 2 mg/mL L-DOPA (L-3,4-dihydroxyphenylalanine) (Sigma-Aldrich, St. Louis, USA) dissolved in PBS was further added. The quantity of dopachrome produced from L-DOPA by the action of tyrosinase was compared by measuring absorbance. After the addition of L-DOPA, absorbance was measured at 475 nm with 10-minute intervals while incubating at 37 °C. Fig. 2 shows a result of calculating the tyrosinase content from the amount of proteins quantitated in (3) of Test Example 1 according to Equation 2.

[Equation 2]

Tyrosinase content (%) = (Absorbance / Total protein) x 100

As seen from Fig. 2, when the cells were treated with the *Scutellaria alpina* extract of the present disclosure, the tyrosinase activity which had been increased by the pigmentation-inducing substance was decreased. In particular, when 5 ppm of the *Scutellaria alpina* extract was treated, the tyrosinase activity decreased to about 99%, comparable to that of the control group.

Accordingly, it was confirmed that the *Scutellaria alpina* extract of the present disclosure exhibits a remarkable effect of decreasing tyrosinase activity and thus it can exhibit the effect of reducing melanin production, whitening skin or treating pigmentation.

Hereinafter, various formulation examples of the composition according to an aspect of the present disclosure will be described. However, other formulations are also possible and the scope of the present disclosure is not limited by the formulation examples.

[Formulation Example 1] Soft capsule

8 mg of the *Scutellaria alpina* extract of Example 1, 9 mg of vitamin E, 9 mg of vitamin C, 2 mg of palm oil, 8 mg of hydrogenated vegetable oil, 4 mg of yellow beeswax and 9 mg of lecithin were mixed according to a commonly employed method. A soft capsule was prepared by filling 400 mg of the mixture per capsule. Separately from this, a soft capsule sheet was prepared from 66 parts by weight of gelatin, 24 parts by weight of glycerin and 10 parts by weight of sorbitol, which was then filled with the mixture to prepare a soft capsule in which 400 mg of the composition according to the present disclosure is contained.

[Formulation Example 2] Tablet

8 mg of the *Scutellaria alpina* extract of Example 1, 9 mg of vitamin E, 9 mg of vitamin C, 200 mg of galactooligosaccharide, 60 mg of lactose and 140 mg of maltose were mixed and granulated using a fluidized-bed dryer. Then, after adding 6 mg of sugar ester, the mixture was prepared into a tablet containing 500 mg of the composition according to the present disclosure.

[Formulation Example 3] Drink

8 mg of the *Scutellaria alpina* extract of Example 1, 9 mg of vitamin E, 9 mg of vitamin C, 10 g of glucose, 0.6 g of citric acid and 25 g of oligosaccharide syrup were mixed and 300 mL of purified water was added. 200 mL of the mixture was filled in a bottle. Then, a drink was prepared by sterilizing at 130 °C for 4-5 seconds.

[Formulation Example 4] Granule

8 mg of the *Scutellaria alpina* extract of Example 1, 9 mg of vitamin E, 9 mg of vitamin C, 250 mg of anhydrous crystalline glucose and 550 mg of starch were mixed and granulated using a fluidized-bed granulator. The resulting granule was filled in a pouch.

[Formulation Example 5] Injection

An injection was prepared according to a commonly employed method with the composition described in Table 2.

[Table 2]

| Ingredient | Content |
|---|---|
| *Scutellaria alpina* extract of Example 1 | 10-50 mg |
| Sterilized distilled water for injection | adequate |
| pH control agent | adequate |

[Formulation Example 6] Health functional food

A health functional food was prepared according to a commonly employed method with the composition described in Table 3.

[Table 3]

| Ingredient | Content |
|---|---|
| *Scutellaria alpina* extract of Example 1 | 20 mg |
| Vitamin A acetate | 70 μg |
| Vitamin E | 1.0 mg |
| Vitamin $B_1$ | 0.13 mg |
| Vitamin $B_2$ | 0.15 mg |
| Vitamin $B_6$ | 0.5 mg |
| Vitamin $B_{12}$ | 0.2 μg |
| Vitamin C | 10 mg |
| Biotin | 10 μg |
| Nicotinamide | 1.7 mg |
| Folic acid | 50 μg |
| Calcium pantothenate | 0.5 mg |
| Ferrous sulfate | 1.75 mg |
| Zinc oxide | 0.82 mg |
| Magnesium carbonate | 25.3 mg |
| Potassium dihydrogen phosphate | 15 mg |
| Calcium monohydrogen phosphate | 55 mg |
| Potassium citrate | 90 mg |
| Calcium carbonate | 100 mg |
| Magnesium chloride | 24.8 mg |

Although specific compositions of vitamin and mineral mixtures suitable for a health functional food were described as above, the compositions may be changed as desired.

[Formulation Example 7] Health drink

A health drink was prepared according to a commonly employed method with the composition described in Table 4.

[Table 4]

| Ingredient | Content |
|---|---|
| *Scutellaria alpina* extract of Example 1 | 1000 mg |

(continued)

| Ingredient | Content |
|---|---|
| Citric acid | 1000 mg |
| Oligosaccharide | 100 g |
| Taurine | 1 g |
| Purified water | balance |

According to a commonly employed health drink preparation method, the above-described ingredients were mixed and heated at 85 °C for about 1 hour under agitation. The resulting solution was filtered and sterilized.

[Formulation Example 8] Softening lotion (skin lotion)

A softening lotion was prepared according to a commonly employed method with the composition described in Table 5.

[Table 5]

| Ingredient | Content (wt%) |
|---|---|
| *Scutellaria alpina* extract of Example 1 | 0.2 |
| Glycerin | 3.0 |
| Butylene glycol | 2.0 |
| Propylene glycol | 2.0 |
| Carboxyvinyl polymer | 0.1 |
| PEG-12 nonyl phenyl ether | 0.2 |
| Polysorbate 80 | 0.4 |
| Ethanol | 10.0 |
| Triethanolamine | 0.1 |
| Preservative, pigment and flavor | adequate |
| Purified water | balance |

[Formulation Example 9] Nourishing lotion (milk lotion)

A nourishing lotion was prepared according to a commonly employed method with the composition described in Table 6.

[Table 6]

| Ingredient | Content (wt%) |
|---|---|
| *Scutellaria alpina* extract of Example 1 | 1.0 |
| Glycerin | 3.0 |
| Butylene glycol | 3.0 |
| Propylene glycol | 3.0 |
| Carboxyvinyl polymer | 0.1 |
| Beeswax | 4.0 |
| Polysorbate 60 | 1.5 |
| Caprylic/capric triglyceride | 5.0 |

(continued)

| Ingredient | Content (wt%) |
|---|---|
| Squalane | 5.0 |
| Sorbitan sesquioleate | 1.5 |
| Liquid paraffin | 0.5 |
| Cetearyl alcohol | 1.0 |
| Triethanolamine | 0.2 |
| Preservative, pigment and flavor | adequate |
| Purified water | balance |

[Formulation Example 10] Nourishing cream

A nourishing cream was prepared according to a commonly employed method with the composition described in Table 7.

[Table 7]

| Ingredient | Content (wt%) |
|---|---|
| *Scutellaria alpina* extract of Example 1 | 2.0 |
| Glycerin | 3.0 |
| Butylene glycol | 3.0 |
| Liquid paraffin | 7.0 |
| β-Glucan | 7.0 |
| Carbomer | 0.1 |
| Caprylic/capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Preservative, pigment and flavor | adequate |
| Purified water | balance |

[Formulation Example 11] Massage cream

A massage cream was prepared according to a commonly employed method with the composition described in Table 8.

[Table 8]

| Ingredient | Content (wt%) |
|---|---|
| *Scutellaria alpina* extract of Example 1 | 2.0 |
| Glycerin | 8.0 |
| Butylene glycol | 4.0 |
| Liquid paraffin | 45.0 |

(continued)

| Ingredient | Content (wt%) |
|---|---|
| β-Glucan | 7.0 |
| Carbomer | 0.1 |
| Caprylic/capric triglyceride | 3.0 |
| Beeswax | 4.0 |
| Cetearyl glucoside | 1.5 |
| Sorbitan sesquioleate | 0.9 |
| Vaseline | 3.0 |
| paraffin | 1.5 |
| Preservative, pigment and flavor | adequate |
| Purified water | balance |

[Formulation Example 12] Pack

A pack was prepared according to a commonly employed method with the composition described in Table 9.

[Table 9]

| Ingredient | Content (wt%) |
|---|---|
| *Scutellaria alpina* extract of Example 1 | 0.2 |
| Glycerin | 4.0 |
| Polyvinyl alcohol | 15.0 |
| Hyaluronic acid extract | 5.0 |
| β-Glucan | 7.0 |
| Allantoin | 0.1 |
| Nonyl phenyl ether | 0.4 |
| Polysorbate 60 | 1.2 |
| Ethanol | 6.0 |
| Preservative, pigment and flavor | adequate |
| Purified water | balance |

Many modifications can be made to adapt a particular situation or material to the teachings of this disclosure without departing from the essential scope thereof. Therefore, it is intended that this disclosure not be limited to the particular exemplary embodiments disclosed as the best mode contemplated for carrying out this disclosure, but that this disclosure will include all embodiments falling within the scope of the appended claims.

[Accession Number]

[0108]

　　Depository institution: Korea Research Institute of Bioscience and Biotechnology
　　Accession number: KCTC12015 BP
　　Date of accession: Sep. 14, 2011

**Claims**

1. Non-therapeutic cosmetic use of a *Scutellaria alpina* extract as an active ingredient for skin whitening, reducing melanin, or suppressing pigmentation.

2. The use according to claim 1, wherein the *Scutellaria alpina* is one or more selected from a group consisting of the leaf, flower, stem and root of the plant *Scutellaria alpina.*

3. The use according to any of claims 1 to 2, wherein the extract is comprised in a composition, wherein the concentration of the *Scutellaria alpina* extract is 1 ppm (w/w) or higher based on the total weight of the composition.

4. The use according to any of claims 1 to 3, wherein the *Scutellaria alpina* extract is an extract of one or more selected from a group consisting of a water, an organic solvent and a mixture thereof.

5. The use according to claim 4, wherein the organic solvent is one or more selected from a group consisting of a $C_1$-$C_6$ lower alcohol, butylene glycol and propylene glycol.

6. The use according to claim 5, wherein the lower alcohol is ethanol.

7. The use according to any of claims 1 to 6, wherein the extract is comprised in a composition, wherein the composition is a food or cosmetic composition.

8. A *Scutellaria alpina* extract for use in reducing melanin or suppressing pigmentation.

9. The extract for use according to claim 8, wherein the *Scutellaria alpina* is one or more selected from a group consisting of the leaf, flower, stem and root of the plant Scutellaria alpina.

10. The extract for use according to any of claims 8 to 9, wherein the extract is comprised in a composition, wherein the concentration of the *Scutellaria alpina* extract is 1 ppm (w/w) or higher based on the total weight of the composition.

11. The extract for use according to any of claims 8 to 10, wherein the *Scutellaria alpina* extract is an extract of one or more selected from a group consisting of a water, an organic solvent and a mixture thereof.

12. The extract for use according to claim 11, wherein the organic solvent is one or more selected from a group consisting of a $C_1$-$C_6$ lower alcohol, butylene glycol and propylene glycol.

13. The extract for use according to claim 12, wherein the lower alcohol is ethanol.

14. The extract for use according to any of claims 8 to 13, wherein the extract is comprised in a composition, wherein the composition is a pharmaceutical or food composition.

**Patentansprüche**

1. Nicht-therapeutische kosmetische Verwendung eines *Scutellaria alpina*-Extrakts als einen Wirkstoff zum Bleichen von Haut, Reduzieren von Melanin oder Unterdrücken von Pigmentierung.

2. Verwendung nach Anspruch 1, wobei das *Scutellaria alpina* eines oder mehrere ist, ausgewählt aus einer Gruppe bestehend aus dem Blatt, der Blüte, dem Stängel und der Wurzel der Pflanze *Scutellaria alpina.*

3. Verwendung nach einem der Ansprüche 1 bis 2, wobei der Extrakt in einer Zusammensetzung beinhaltet ist, wobei die Konzentration des *Scutellaria alpina*-Extrakts, basierend auf dem Gesamtgewicht der Zusammensetzung, 1 ppm (w/w) oder mehr beträgt.

4. Verwendung nach einem der Ansprüche 1 bis 3, wobei der *Scutellaria alpina*-Extrakt ein Extrakt aus einem oder mehreren ist, ausgewählt aus einer Gruppe bestehend aus einem Wasser, einem organischen Lösungsmittel und einer Mischung davon.

**5.** Verwendung nach Anspruch 4, wobei das organische Lösungsmittel eines oder mehrere ist, ausgewählt aus einer Gruppe bestehend aus einem niederen $C_1$-$C_6$-Alkohol, Butylenglykol und Propylenglykol.

**6.** Verwendung nach Anspruch 5, wobei der niedere Alkohol Ethanol ist.

**7.** Verwendung nach einem der Ansprüche 1 bis 6, wobei der Extrakt in einer Zusammensetzung beinhaltet ist, wobei die Zusammensetzung eine Nahrungsmittel- oder kosmetische Zusammensetzung ist.

**8.** *Scutellaria alpina*-Extrakt zur Verwendung beim Reduzieren von Melanin oder Unterdrücken von Pigmentierung.

**9.** Extrakt zur Verwendung nach Anspruch 8, wobei das *Scutellaria alpina* eines oder mehrere ist, ausgewählt aus einer Gruppe bestehend aus dem Blatt, der Blüte, dem Stängel und der Wurzel der Pflanze *Scutellaria alpina.*

**10.** Extrakt zur Verwendung nach einem der Ansprüche 8 bis 9, wobei der Extrakt in einer Zusammensetzung beinhaltet ist, wobei die Konzentration des *Scutellaria alpina*-Extrakts, basierend auf dem Gesamtgewicht der Zusammensetzung, 1 ppm (w/w) oder mehr beträgt.

**11.** Extrakt zur Verwendung nach einem der Ansprüche 8 bis 10, wobei der *Scutellaria alpina*-Extrakt ein Extrakt aus einem oder mehreren ist, ausgewählt aus einer Gruppe bestehend aus einem Wasser, einem organischen Lösungsmittel und einer Mischung davon.

**12.** Extrakt zur Verwendung nach Anspruch 11, wobei das organische Lösungsmittel eines oder mehrere ist, ausgewählt aus einer Gruppe bestehend aus einem niederen $C_1$-$C_6$-Alkohol, Butylenglykol und Propylenglykol.

**13.** Extrakt zur Verwendung nach Anspruch 12, wobei der niedere Alkohol Ethanol ist.

**14.** Extrakt zur Verwendung nach einem der Ansprüche 8 bis 13, wobei der Extrakt in einer Zusammensetzung beinhaltet ist, wobei die Zusammensetzung eine pharmazeutische oder Nahrungsmittelzusammensetzung ist.

**Revendications**

**1.** Utilisation cosmétique non thérapeutique d'un extrait de *Scutellaria alpina* comme principe actif pour le blanchiment de la peau, la réduction de la mélanine ou la suppression de la pigmentation.

**2.** Utilisation selon la revendication 1, dans laquelle *Scutellaria alpina* est un ou plusieurs élément(s) choisi(s) dans un groupe constitué par la feuille, la fleur, la tige et la racine de la plante *Scutellaria alpina.*

**3.** Utilisation selon l'une des revendications 1 et 2, dans laquelle l'extrait est compris dans une composition, dans laquelle la concentration de l'extrait de *Scutellaria alpina* est supérieure ou égale à 1 ppm (p/p) par rapport au poids total de la composition.

**4.** Utilisation selon l'une des revendications 1 à 3, dans laquelle l'extrait de *Scutellaria alpina* est un extrait d'un ou de plusieurs élément(s) choisi(s) dans un groupe constitué par de l'eau, un solvant organique et un mélange de ceux-ci.

**5.** Utilisation selon la revendication 4, dans laquelle le solvant organique est un ou plusieurs solvant(s) choisi(s) dans un groupe constitué par un alcool inférieur en $C_1$ à $C_6$, le butylène glycol et le propylène glycol.

**6.** Utilisation selon la revendication 5, dans laquelle l'alcool inférieur est l'éthanol.

**7.** Utilisation selon l'une des revendications 1 à 6, dans laquelle l'extrait est compris dans une composition, dans laquelle la composition est une composition alimentaire ou cosmétique.

**8.** Extrait de *Scutellaria alpina* pour une utilisation dans la réduction de la mélanine ou la suppression de la pigmentation.

**9.** Extrait pour une utilisation selon la revendication 8, dans lequel *Scutellaria alpina* est un ou plusieurs élément(s) choisi(s) dans un groupe constitué par la feuille, la fleur, la tige et la racine de la plante *Scutellaria alpina.*

**10.** Extrait pour une utilisation selon l'une des revendications 8 et 9, dans lequel l'extrait est compris dans une composition, dans lequel la concentration de l'extrait de *Scutellaria alpina* est supérieure ou égale à 1 ppm (p/p) par rapport au poids total de la composition.

**11.** Extrait pour une utilisation selon l'une des revendications 8 à 10, dans lequel l'extrait de *Scutellaria alpina* est un extrait d'un ou de plusieurs élément(s) choisi(s) dans un groupe constitué par de l'eau, un solvant organique et un mélange de ceux-ci.

**12.** Extrait pour une utilisation selon la revendication 11, dans lequel le solvant organique est un ou plusieurs solvant(s) choisi(s) dans un groupe constitué par un alcool inférieur en $C_1$ à $C_6$, le butylène glycol et le propylène glycol.

**13.** Extrait pour une utilisation selon la revendication 12, dans lequel l'alcool inférieur est l'éthanol.

**14.** Extrait pour une utilisation selon l'une des revendications 8 à 13, dans lequel l'extrait est compris dans une composition, dans lequel la composition est une composition pharmaceutique ou alimentaire.

【Fig. 1】

【Fig. 2】

(*p<0.05,**p<0.01)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101111533 B1
- KR 2012007815 W
- KR 1020110099728

**Non-patent literature cited in the description**

- *Korean Collection for Type Culture (KCTC) of Korea Research Institute of Bioscience and Biotechnology,* 14 September 2011